(19) Europäisches Patentamt
European Patent Office
Office européen des brevets

(11) **EP 2 420 289 A1**

(12) **EUROPEAN PATENT APPLICATION**

(43) Date of publication:
**22.02.2012 Bulletin 2012/08**

(51) Int Cl.:
***A61P 35/00*** (2006.01)       ***A61K 31/216*** (2006.01)
***A61K 31/353*** (2006.01)

(21) Application number: **10173255.0**

(22) Date of filing: **18.08.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Grindeks, a joint stock company**
**1057 Riga (LV)**

(72) Inventors:
• **Kalvins, Ivars**
**Riga LV-1006 (LV)**

• **Ilmars, Stonans**
**Riga LV-1006 (LV)**
• **Sestakova, Irina**
**Riga LV-1006 (LV)**
• **Domracova, Ilona**
**Riga LV-1006 (LV)**
• **Jascenko, Elina**
**Riga LV-1006 (LV)**
• **Bridane, Veronika**
**Riga LV-1006 (LV)**
• **Kanepe, Iveta**
**Riga LV-1006 (LV)**

(54) **Composition of caffeic acid phenethyl ester (CAPE) and one of catechin, kaempherol and myricetin for potentiating antitumour effect and for treating tumours**

(57) Novel pharmaceutical composition of treating cancer, preferably malignant cancer by way of enteral use have been described, which contain Caffeic acid phenethyl ester and one of natural flavonoid derivative, preferably (+)-Catechin, Myricetin, Kaempferol.

EP 2 420 289 A1

**Description**

Technical Field

The present invention relates to a novel composition of caffeic acid phenethyl ester and natural flavonoid derivatives; in particular, it relates to the unexpected synergism of a combination of caffeic acid phenethyl ester (CAPE) and one of natural flavonoid derivative in the treatment of tumours; and to processes for preparing the composition and its use for chemotherapy of tumours, especially malignant tumours.

Background Art

More than 11 million people are diagnosed with cancer every year. It is estimated that there will be 16 million new cases every year by 2020. Cancer causes 7 million deaths every year - or 12.5% of deaths worldwide.

Therefore, a great number of investigations, developments and research work have been carried out to find anticancer agents, which can be used in chemotherapy of malignant tumours. There are known various anticancer agents and results of cancer treatment improve with every year, nevertheless the amount of effective dose is still high.

In nowadays a big role in anticancer experiments and in treating cancer take natural compounds. Phytochemicals are one of them, which are naturally occurring anticancer agents.

Phytochemicals are naturally occurring biochemicals that plants developed in order to protect themselves from oxidation, insects, disease organisms and other hazards in their environment, published in http://www.suite101.com/ article.cfm/new_cancer _treatments/104912 18.07.2007

There are more than thousand phytochemicals. Some of such are phenolic compound from propolis - Caffeic acid phenethyl ester, and natural flavonoid derivatives such as Kaempferol, Myricetin, Catechin.

Caffeic acid phenethyl ester (CAPE) is a phenolic compound contained in propolis, which is the generic name of a resinous product derived from the bark of conifer trees and carried out by honeybees to their hives (see CHIAO, C. "Apoptosis and altered redox state induced by caffeic acid phenethyl ester (CAPE) in transformed rat fibrolast cells", published in "Cancer Research" 1995, vol.55, no 3576, p.3583). The biological activities of propolis and CAPE have been studied, and it has been shown that CAPE also has an antitumour activity. Various investigators have demonstrated that CAPE furthermore has anti-inflammatory properties both in vitro and in vivo (see MIRZOEVA, O., "The effect of propolis and its components on eicosanoid production during the inflammatory response", published in "Prostaglandins Leukotrienes Essent Fatty Acids" 1996, p.441-449, and ORBAN, Z. "Caffeic acid phenethyl ester induces leukocyte apoptosis. Modulates nuclear factor- {kappa} B and suppresses acute inflammation", published in "Neuroimmunomodul" 2000, vol.7, p.99-105). CAPE has also been proposed as a specific inhibitor of the transcription factor nuclear factor - {kappa} B, which may account for its anti-inflammatory actions. Specifically, in a human histiocytic cell line, CAPE only inhibited NF- {kappa} B, as opposed to other transcription factors, such as API, TFIID, and Oct-1. In this regard, it has been proposed to use CAPE directly for inhibiting the interaction of NF- K 13 with DNA (see NATARAJAN, K. et al., "Caffeic acid phenethyl ester is a potent and specific inhibitor of activation of nuclear transcription factor NF-{kappa} B, published in "Immunology", 1996, vol.93, p.9090-9095). CAPE also clearly induces apoptosis in various cell types.

US 5008441 (UNIV COLUMBIA, published 16.04.1991) discloses a method for producing caffeic acid phenenthyl ester (CAPE) and a method which substantially inhibits the growth of normal cells. This process comprises treating a population of cells with an effective inhibiting amount of caffeic acid phenethyl ester (CAPE) so as to substantially inhibit the growth of transformed cells. Transformed cells are human breast carcinoma cells, melanoma cells (SK-MEL-28 or SK-MEKL-1 70) or colon or renal carcinoma cells. In MASAHIKO, W., et al., "Caffeic Acid Phenethyl Ester Induces Apoptosis by Inhibition of NF {kappa} B and Activation of Fas in Human Breast Cancer MCF-7 Cells", published in "Journal of Biological Chemistry" 2004, vol.279, no7, 0.6017-6026, caffeic acid phenethyl ester is described as a flavonoid derivative, which is a biologically active ingredient of honeybee propolis and strongly inhibits NF {kappa} B activation. Furthermore, it was demonstrated that apoptosis is induced by CAPE in various cancer cell lines - e.g. human breast cancer MCF-7 cells. It was found by TAKEMA, N., et al., in "Selective antiproliferative activity of caffeic acid phenethyl ester analogues on highly liver-metastatic murine colon 26-L5 carcinoma cell line", published in "Bioorganic & Medicinal Chemistry 2002, vol. 10, no. 10, p.3351-3359, that CAPE possesses selective antiproliferative activity toward a highly liver-metastatic murine colon 26-L5 carcinoma cell line.

Flavonoids are polyphenolic secondary metabolites widely dispersed throughout the plant kingdom and found in substantial levels in commonly consumed fruits, vegetables and beverages. Flavonoids, which are benzo-{gamma}-pyrone derivates with A, B and C rings, are categorized as:

flavonols, flavones, flavan-3-ols, isoflavones, flavonones and
anthocyanidins. They have been shown to possess a variety of biological activity at non-toxic concentrations in organisms. The role of dietary flavonoids in cancer prevention is widely discussed. Compelling data from laboratory studies, epidemiological investigations, and human clinical trials indicate that flavonoids have important effect on cancer chemoprevention and chemotherapy. Many mechanisms of action have been identified, including carcinogen inactivation, antiproliferation, cell cycle arrest, induction of apoptosis and differentiation, inhibition of angiogenesis, antioxidation and reversal of multidrug resistance or a combination of these mechanisms. Based on these results, flavonoids may be promising anticancer agents, see (REN W., QIAO Z., "Flavonoids: Promising anticancer agents", published in "Medical Research Reviews" 2003, vol. 23., no 4., p. 519-534).

(+)-Catechin (Ca) is a class of flavonoids with potent antioxidant and cancer chemopreventive properties. These compounds are found in a variety of plants and are present in particularly high amounts in tea leaves, where they may constitute up to 30% of dry leaf weight. High levels of monomeric (+)-catechin are found in the skin and seeds of fruits such as apples and grapes. In keeping with ability of phenolic antioxidants to induce the expression of phase II detoxifying enzymes in mammalian cells,
(+)-catechin produces antimutagenic effects, see (MICHAEL J., "(+)-Catechins Inhibits Intestinal Tumor Formation and Suppresses Focal Adhesion Kinase Activation in the Min/+ Mouse", published in "Cancer Research" 2001, vol. 61., no. 1., p. 118-125).

Myricetin is a naturally-occurring flavonoid in many grapes, berries, fruits, vegetables, herbs, as well as other plants. Myricetin is considered to be an antioxidant, which means that it is capable eliminating cancer causing free radicals within the body, published in http://www.nutritional-supplement-guides.com/Myricetin.html 25.07.2007

There are data that Myricetin exhibits a significant induction of differentiation in the human osteoblast-like cell line MG-63, and Myricetin affects inflammatory cytokines-mediated apoptosis in osteoblast cells. Treatment of MG-63 cells with myricetin not only inhibited anti-Fas IgM-induced apoptosis, but also blocked the synergetic effect of anti-Fas IgM with Tumor Necrosis factor-alpha or IL-1beta on cell death, see (KUO PL, "Myricetin inhibits the induction of anti-Fas IgM-, tumor necrosis factor-alpha- and interleukin-1-beta-mediated apoptosis by Fas pathway inhibition in human osteoblastic cell line MG-63.", published in "Life Sciences" 2005, vol.77., no.23., p. 2964-76.).

Kaempferol is a natural flavonoid which has been isolated from tea, broccoli, Delphinium, grapefruit, and other plant sources. Kaempferol is a strong antioxidant and helps to prevent oxidative damage of our cells, lipids and DNA. Studies have also confirmed that kaempferol acts as a chemopreventive agent, which means that it inhibits the formation of cancer cells, published in

http://www.phytochemicals.info/phytochemicals/kaempferol.php 26.07.2007

Prior art discloses that there are data of two flavonoids: kaempferol and quercetin, acting synergistically. Studies shows that kaempferol act synergistically in reducing cell proliferation of cancer cells, meaning that the combined treatments with quercetin and kaempferol are more effective that the additive effects of each other, see (ACKLAND ML "Synergistic antiproliferative action of the flavonols quercetin and kaempferol in cultured human cell lines", published in "In Vivo" 2005, vol.19., no. 1., p.69-76.)

Caffeic acid phenethyl ester is a flavonoid like compound and its chemical structure totally differs from Qyercetin's chemical structure.

WO 2005004630 discloses a composition of Resveratrol, Catechin and Caffeic acid for pharmaceutical use in an oral or topical way, treating disorders from oxidative stress, and tumours are one of these.

Caffeic acid phenethyl ester is a derivative from Caffeic acid.

Disclosure of Invention

Technical problem

In malignant tumour treatment different kind of pharmaceutical compositions are used, including compositions of different natural compounds, but just only partial remission of malignant tumour was obtained.

The further aim of present invention is to developed effective pharmaceutical composition that can be used in malignant tumour treatment for achieving increased remission of tumours.

Technical solution

The present invention is directed to a composition for treating a tumour, especially a malignant tumour in an individual who need such treatment, comprising the administration to said individual of a pharmacologically effective dose of one or more natural flavonoid derivatives, especially CAPE and Catechin, Myricetin, Kaempferol.

While attempting to develop a pharmaceutical composition for malignant tumour treatment having an essentially lower toxicity, we unexpectedly found that doses adequate to the therapeutic ones used in the clinical use of pharmaceutical compositions containing Caffeic acid phenethyl ester, the co-administration or combined use in pharmaceutical compositions of a compound of a natural flavonoid derivatives, preferably (+)-Catechin, Myricetin, Kaempferol leads to advantageous effects, such as:

■ Combination of caffeic acid phenethyl ester (CAPE) and natural flavonoid derivative, preferably (+)-Catechin, Kaempferol, Myricetin showed remarkable synergism between the components in the treatment of tumour; ■ The use of a combination of CAPE and natural flavonoid derivative, preferably (+)-Catechin, Myricetin, Kaempferol, that allows efficacy increase growth inhibition of S-180 ascites tumour.

There is no particular restriction on the unit dosage from which can be adopted the antitumour composition of the invention in the treatment of malignant tumours in mammals. The unit dosage form is selected according to the purpose of treatment. Examples thereof are oral dosage form such as tablets, coated tablets, pills, powders, granules, capsules, solutions, suspensions, emulsions, etc. and parenteral dosage forms such as suppositories, ointments, plasters, etc. These dosage forms can be manufactured by conventional pharmaceutical procedures known in this field.

As the carrier for shaping into the form of tablets, there can be employed various excipients such as lactose, sucrose, sodium chloride, glucose, urea, starch, calcium carbonate, kaolin, crystalline cellulose, silicic acid, etc.; binders such as simple syrup, glucose solution, starch solution, gelatine solution, carboxymethylcellulose, shellac, methylcellulose, potassium phosphate, polyvinylpyrrolidone, etc.; disintegrators such as fry starch, sodium alginate, agar powder, laminaran powder, sodium hydrogen carbonate, calcium carbonate, polyoxyethylene-sorbitan fatty acid esters, sodium lauryl sulphate, stearic acid monoglyceride, starch, lactose, etc.; antidisintegrators such as sucrose, stearic acid, cacao butter, hydrogenated oil, etc.; absorption promoters such as quaternary ammonium bases, sodium lauryl sulfate, etc.; humectants such as glycerol, starch, etc.; adsorbents such as starch, lactose, kaolin, bentonite, colloidal silicic acid, etc.; and lubricants such as purified talc, stearic acid salts, boric acid powder, polyethylene glycol, etc. Where necessary, the tablets may be in the form of coated tablets such as sugar-coated tablets, gelatin-coated tablets, enteric-coated tablets, film-coated tablets, or double or multi-layer tablets, etc.

The carrier for shaping into the form of pills includes, for example, various excipients such as glucose, lactose, starch, cacao butter, hardened vegetable oil, kaolin, talc, etc.; binders such as gum arabic powder, gum tragacanth powder, gelatine, etc.; and disintegrators such as laminaran, agar, etc. The carrier for shaping into the form of suppositories includes, for example, polyethylene glycol, cacao butter, higher alchols, esters of higher alcohols, gelatine, semi-synthetic glycerides, etc.

Capsules are manufactured by mixing the natural flavonoid derivative (CAPE) with one of natural flavonoid derivative, preferably (+)-Catechin, Kaempferol, Myricetin, with any of the carriers mentioned above and encapsulating the mixture in hard gelatine capsule, soft capsule or other capsules.

For manufacturing in the form of pastes, creams and gels, there is employed a diluents such as, for example, white petrolatum, paraffin, glycerol, cellulose derivatives, polyethylene glycols, silicone, bentonite, etc.


Best Mode for Carrying Out the Invention

The present invention in the following will be described in more derail with reference to pharmacological tests and examples illustrating the preparation of the anti-tumour effect-potentiating compositions of the invention comprising caffeic acid phenethyl ester and natural flavonoid derivatives, preferably Catechin, Kaempferol or Myricetin .


Mode(s) for Carrying Out the Invention

Experimental example

Experiments in vivo were carried out on mice sarcoma S-180 ascites form.

The anti-tumour activity of the pharmaceutical composition of caffeic acid phenethyl ester (CAPE) and one of natural flavonoid derivative, preferably Catechin, Kaempferol or Myricetin was determined by introducing them into ICR mice of initial weight 20-23 g during a fortnight. Throughout the experiment, the mice were kept under standard laboratory conditions at a temperature of $22 \pm 2$ °C, relative humidity $55 \pm 15\%$, ventilation - 15-20 changes of air amount/hour and a 12 hours light/darkness cycle, and were fed with standard laboratory animal food.

Mice sarcoma S-180 transplanted intraperitoneally to ICR mice in amount 5 x $10^6$ cell/mouse.

Then, 24 hours later, orally administration of compounds was started once a day at the 1st, 3rd, 5th, 7th, 9th, 11th and 13th days.

The compound efficacy was expressed as the percentage of growth inhibition of S-180 ascites tumour, calculated using the equation:

Growth inhibition of S - 180 ascites tumour(%) $= 100 - \left( \dfrac{T}{C} \times 100 \right)$

wherein C - mean weight (g) of the control group and T - mean weight (g) of the experimental group.

The results of Table 1 show the growth inhibition of S-180 ascites tumour at 13th day after the transplantation of tumour in mice.

Table 1

| Nr. | Drug | Dosage (mg/kg) | Growth inhibition of S-180 ascites tumour, % |
|---|---|---|---|
| 1 | CAPE | 9 | -2 |
| 2 | Catechin | 100 | 9 |
| 3 | CAPE+ Catechin | 9+100 | 41 |
| 4 | CAPE | 100 | 35 |
| 5 | Kaempferol | 200 | 29 |
| 6 | CAPE+ Kaempferol | 100+200 | 72 |
| 7 | CAPE | 50 | 24 |
| 8 | Myricetin | 50 | -9 |
| 9 | CAPE+ Myricetin | 50+50 | 39 |

As it is shown in Table 1 combinations of CAPE and one of natural flavonoid derivative showed remarkable synergism between the components and allows efficacy increase growth inhibition of S-180 ascites tumour.
Formulation: Granules

Table 1

| | Example 1 | Example 2 | Example 3 | Example 4 |
|---|---|---|---|---|
| CAPE | 400 mg | 200 mg | 400 mg | 20 mg |
| Catechin | 400 mg | 0 | 0 | 0 |
| Kaempferol | 0 | 400 mg | 0 | 200 mg |
| Myricetin | 0 | 0 | 100 mg | 0 |
| Lactose | 200 mg | 200 mg | 200 mg | 200 mg |
| Corn starch | 200 mg | 200 mg | 200 mg | 200 mg |
| Hydroxumethyl-cellulose | 10 mg | 10 mg | 10 mg | 10 mg |
| Total | 1210 mg | 1010 mg | 910 mg | 630 mg |

Using the conventional procedure, the granules were prepared according to the above formula.
Formulation: Tablets

| | Example 1 | Example 2 | Example 3 | Example 4 |
|---|---|---|---|---|
| CAPE | 50 mg | 100 mg | 10 mg | 100 mg |
| Catechin | 50 mg | 0 | 0 | 50 mg |
| Kaempferol | 0 | 0 | 120 mg | 0 |
| Myricetin | 0 | 10 mg | 0 | 0 |
| Lactose | 100 mg | 100 mg | 100 mg | 100 mg |
| Crystalline cellulose | 50 mg | 50 mg | 50 mg | 50 mg |
| Magnesium stearate | 10 mg | 10 mg | 10 mg | 10 mg |
| Talc | 10 mg | 10 mg | 10 mg | 10 mg |
| Total | 270 mg | 280 mg | 300 mg | 320 mg |

Using the conventional procedure, the tablets were prepared according to the above formula.

Formulation: Capsules

|  | Example 1 | Example 2 | Example 3 | Example 4 |
|---|---|---|---|---|
| CAPE | 90 mg | 100 mg | 10 mg | 50 mg |
| Catechin | 30 mg | 0 | 0 | 200 mg |
| Kaempferol | 0 | 50 mg | 0 | 0 |
| Myricetin | 0 | 0 | 110 mg | 0 |
| Lactose | 100 mg | 100 mg | 100 mg | 150 mg |
| Talc | 100 mg | 100 mg | 100 mg | 150 mg |
| Ca stearate | 7 mg | 7 mg | 7 mg | 7 mg |
| Aerosil | 3 mg | 3 mg | 3 mg | 5 mg |
| Total | 330 mg | 360 mg | 330 mg | 560 mg |

Using the conventional procedure, the capsules were prepared according to the above formula.

Formulation: Suppository

|  | Example 1 | Example 2 | Example 3 | Example 4 |
|---|---|---|---|---|
| CAPE | 50 mg | 200 mg | 100 mg | 20 mg |
| Catechin | 150 mg | 0 | 0 | 200 mg |
| Kaempferol | 0 | 100 mg | 0 | 0 |
| Myricetin | 0 | 0 | 100 mg | 0 |
| Witepsol W-35 | 350 mg | 400 mg | 300 mg | 380 mg |
| Total | 500 mg | 700 mg | 500 mg | 600 mg |

Using the conventional procedure, the suppositories were prepared according to the above formula.

Formulation: Suspension

|  | Example 1 | Example 2 | Example 3 | Example 4 |
|---|---|---|---|---|
| CAPE | 150 mg | 200 mg | 100 mg | 180 mg |
| Catechin | 150 mg | 0 | 0 | 0 |
| Kaempferol | 0 | 50 mg | 0 | 90 mg |
| Myricetin | 0 | 0 | 200 mg | 0 |
| Glycerin | 100 mg | 100 mg | 100 mg | 100 mg |
| Disstilled water | 200 mg | 150 mg | 200 mg | 150 mg |
| Total | 600 mg | 500 mg | 600 mg | 520 mg |

Using the conventional procedure, the suppositories were prepared according to the above formula.

Industrial Application

According to the invention, the pharmaceutical composition for treating malignant tumour which contains Caffeic acid phenethyl ester and one of natural flavonoid derivative, preferably (+)-Catechin, Kaempferol, Myricetin in clinically efficacious amount might be manufactured in a standard pharmaceutical plant.

**Claims**

1. A pharmaceutical composition comprising Caffeic acid phenethyl ester (CAPE) or a pharmaceutically acceptable salt thereof and one of natural flavonoid derivative selected from the group of (+)-Catechin, Kaempferol or Myricetin.

2. A pharmaceutical composition according to claim 1, wherein one of natural flavonoid derivative is (+)-Catechin.

3. A pharmaceutical composition according to claim 1, wherein one of natural flavonoid derivative is Kaempferol.

4. A pharmaceutical composition according to claim 1, wherein one of natural flavonoid derivative is Myricetin.

5. A pharmaceutical composition according to claim 1 the preferable ratio of the active ingredients caffeic acid phenethyl ester (CAPE) and one of natural flavonoid derivative selected from the group of (+)-Catechin, Kaempferol or Myricetin is from 1:100 to 100:1.

6. A pharmaceutical composition according to claim 1 the preferable ratio of the active ingredients caffeic acid phenethyl ester (CAPE) and one of natural flavonoid derivative selected from the group of (+)-Catechin, Kaempferol or Myricetin is from 1:20 to 20:1.

7. A pharmaceutical composition according to claim 1 the preferable ratio of the active ingredients caffeic acid phenethyl ester (CAPE) and one of natural flavonoid derivative selected from the group of (+)-Catechin, Kaempferol or Myricetin is from 2:1 to 1:2.

8. Use of pharmaceutical composition according to any preceding claim as a medicament.

9. A pharmaceutical composition according to claims 1-7 for use in the treatment of a tumour.

10. Use of pharmaceutical composition according to claims 1-7 for the manufacture of a medicament for administration simultaneously, sequentially or separately for treatment and/or prevention of tumour.

Europäisches Patentamt

European Patent Office

Office européen des brevets

## EUROPEAN SEARCH REPORT

Application Number

EP 10 17 3255

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/78783 A (HAUSER INC [US]; UNIV TEXAS [US]) 25 October 2001 (2001-10-25) | 1,3,5-10 | INV. A61P35/00 A61K31/216 A61K31/353 |
| Y | * page 14, line 15 - page 18, line 13; table 2 * | 2,4,9,10 | |
| X | EP 1 417 968 A (UNI DEGLI STUDI DI CATANIA [IT]) 12 May 2004 (2004-05-12) | 1,3,5-10 | |
| Y | * paragraph [0014] * <br> * paragraph [0019]; claims 1-8 * | 2,4 | |
| X | GARDANA ET AL: "Analysis of the polyphenolic fraction of propolis from different sources by liquid chromatography-tandem mass spectrometry" JOURNAL OF PHARMACEUTICAL AND BIOMEDICAL ANALYSIS, NEW YORK, NY, US, vol. 45, no. 3, 19 October 2007 (2007-10-19), pages 390-399, XP022306730 ISSN: 0731-7085 | 1,3,5-10 | |
| Y | * page 390, left-hand column, line 10 - right-hand column, line 3; table 2 * | 2,4 | TECHNICAL FIELDS SEARCHED (IPC) <br> A61K A61P |
| X | MOHAMMADZADEH ET AL: "Chemical composition, oral toxicity and antimicrobial activity of Iranian propolis" FOOD CHEMISTRY, ELSEVIER SCIENCE PUBLISHERS LTD, GB, vol. 103, no. 4, 27 March 2007 (2007-03-27), pages 1097-1103, XP022003107 ISSN: 0308-8146 | 1,3,5-8 | |
| Y | * page 1099, left-hand column; table 1 * | 2,4,9,10 | |

-/--

The present search report has been drawn up for all claims

| Place of search | Date of completion of the search | Examiner |
|---|---|---|
| Munich | 10 November 2010 | Herrera, Suzanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 17 3255

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ORSOLIC N ET AL: "Effects of local administration of propolis and its polyphenolic compounds on tumor formation and growth" BIOLOGICAL AND PHARMACEUTICAL BULLETIN 200510 JP, vol. 28, no. 10, October 2005 (2005-10), pages 1928-1933, XP002505055 | 1,3,5-10 | |
| Y | * the whole document * | 2,4 | |

TECHNICAL FIELDS
SEARCHED        (IPC)

The present search report has been drawn up for all claims

| Place of search | Date of completion of the search | Examiner |
|---|---|---|
| Munich | 10 November 2010 | Herrera, Suzanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

EP 2 420 289 A1

**ANNEX TO THE EUROPEAN SEARCH REPORT
ON EUROPEAN PATENT APPLICATION NO.**                  EP 10 17 3255

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2010

| Patent document cited in search report | | Publication date | Patent family member(s) | | Publication date |
|---|---|---|---|---|---|
| WO 0178783 | A | 25-10-2001 | AU | 5160201 A | 30-10-2001 |
| EP 1417968 | A | 12-05-2004 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5008441 A
- WO 2005004630 A

### Non-patent literature cited in the description

- **CHIAO, C.** Apoptosis and altered redox state induced by caffeic acid phenethyl ester (CAPE) in transformed rat fibrolast cells. *Cancer Research,* 1995, vol. 55 (3576), 3583
- **MIRZOEVA, O.** The effect of propolis and its components on eicosanoid production during the inflammatory response. *Prostaglandins Leukotrienes Essent Fatty Acids,* 1996, 441-449
- **ORBAN, Z.** Caffeic acid phenethyl ester induces leukocyte apoptosis. Modulates nuclear factor- {kappa} B and suppresses acute inflammation. *Neuroimmunomodul,* 2000, vol. 7, 99-105
- **NATARAJAN, K. et al.** Caffeic acid phenethyl ester is a potent and specific inhibitor of activation of nuclear transcription factor NF-{kappa} B. *Immunology,* 1996, vol. 93, 9090-9095
- **MASAHIKO, W. et al.** Caffeic Acid Phenethyl Ester Induces Apoptosis by Inhibition of NF {kappa} B and Activation of Fas in Human Breast Cancer MCF-7 Cells. *Journal of Biological Chemistry,* 2004, vol. 279 (7), 0.6017-6026
- **TAKEMA, N. et al.** Selective antiproliferative activity of caffeic acid phenethyl ester analogues on highly liver-metastatic murine colon 26-L5 carcinoma cell line. *Bioorganic & Medicinal Chemistry,* 2002, vol. 10 (10), 3351-3359
- **REN W. ; QIAO Z.** Flavonoids: Promising anticancer agents. *Medical Research Reviews,* 2003, vol. 23 (4), 519-534
- **MICHAEL J.** +)-Catechins Inhibits Intestinal Tumor Formation and Suppresses Focal Adhesion Kinase Activation in the Min/+ Mouse. *Cancer Research,* 2001, vol. 61 (1), 118-125
- **KUO PL.** Myricetin inhibits the induction of anti-Fas IgM-, tumor necrosis factor-alpha- and interleukin-1-beta-mediated apoptosis by Fas pathway inhibition in human osteoblastic cell line MG-63. *Life Sciences,* 2005, vol. 77 (23), 2964-76
- **ACKLAND ML.** Synergistic antiproliferative action of the flavonols quercetin and kaempferol in cultured human cell lines. *In Vivo,* 2005, vol. 19 (1), 69-76